(19) Europäisches Patentamt / European Patent Office / Office européen des brevets

(11) **EP 3 056 572 A1**

(12) **EUROPEAN PATENT APPLICATION**

(43) Date of publication:
**17.08.2016 Bulletin 2016/33**

(51) Int Cl.:
***C12Q 1/68*** (2006.01)

(21) Application number: **15203179.5**

(22) Date of filing: **30.10.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **30.10.2007 US 983737 P**

(62) Document number(s) of the earlier application(s) in accordance with Art. 76 EPC:
**08019010.1 / 2 055 787**

(71) Applicant: **Applied Biosystems, LLC
Carlsbad, CA 92008 (US)**

(72) Inventors:
• **Hennessy, Lori K.
San Mateo, CA 94403 (US)**
• **Green, Robert
Cupertino, CA 95014 (US)**

(74) Representative: **Grund, Martin
Grund
Intellectual Property Group
Postfach 44 05 16
80754 München (DE)**

Remarks:
•This application was filed on 30-12-2015 as a divisional application to the application mentioned under INID code 62.
•Claims filed after the date of filing of the application/after the date of receipt of the divisional application (Rule 68(4) EPC).

(54) **METHODS AND KITS FOR MULTIPLEX AMPLIFICATION OF SHORT TANDEM REPEAT LOCI**

(57) Methods and materials are disclosed for use in simultaneously amplifying at least 11 specific STR loci of genomic DNA in a single multiplex reaction, as are methods and materials for use in the analysis of the products of such reactions. Included in the present invention are materials and methods for the simultaneous amplification of 16 specific loci in a single multiplex reaction, comprising the 10 AmpFISTR® SGMplus® STR loci, the Amelogenin locus, and 5 new STR loci, including methods and materials for the analysis of these loci.

EP 3 056 572 A1

**Description**

The present teachings are generally directed to the detection of genetic markers in a genomic system. In various embodiments, multiple distinct polymorphic genetic loci are simultaneously amplified in one multiplex reaction in order to determine the alleles of each locus. The polymorphic genetic loci analyzed may be short tandem repeat (STR) loci, which can also include mini-STRs which produce amplicons of approximately 200 base pairs or fewer.

**FIGURES**

The skilled artisan will understand that the figures, described below, are for illustration purposes only. The figures are not intended to limit the scope of the present teachings in any way.

F1G. 1 is a plot which demonstrates the relative size ranges of the amplicons (in base pairs) as produced by multiplex amplification of fifteen STR loci (the ten SGMplus® loci plus five new loci) and the Amelogenin sex determination locus (Amel), as described in the Example.

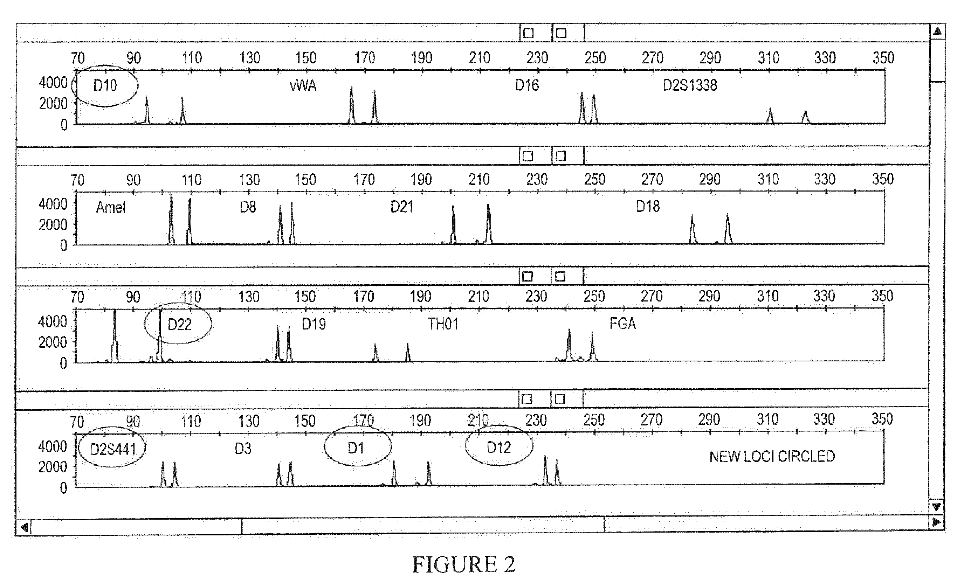

FIG. 2 is a plot of the output from five-color fluorescent detection of the products of simultaneous amplification of the SGMplus® STR loci VWA (vWA), D16S539 (D16), D2S1338, D8S1179 (D8), D2IS11 (D21), D18S51 (D18), D19S433 (D19), TH01, FGA, D3S1358 (D3), the sex determination locus Amelogenin (Amel), and five new STR loci (circled) D10SI248 (D10), D22S1045 (D22), D2S441, D1S1656 (D1) and D12S391 (D12). Loci were amplified from a sample of human genomic DNA and detected with the ABI PRISM® 3130 x1 genetic analyzer, as described in the Example. The four panels correspond, from top to bottom, to 6-FAM™, VIC®, NED™ and PET® dye labeled peaks (the fifth dye, LIZ™, was used to label size standards, and is not shown). The x-axis of each panel measures the size of the amplification product in base pairs.

FIG. 3 is a plot of the emission spectra (wavelengths in nm) of the five fluorescent dyes as used in the Example (6-FAM™, VIC®, NED™, PET® and LIZ™), plus an additional sixth dye (SID) that could be used in a six-dye multiplex reaction.

**DESCRIPTION OF VARIOUS EMBODIMENTS**

Most of the words used in this specification have the meaning that would be attributed to those words by one skilled in the art. Words specifically defined in the specification have the meaning provided in the context of the present teachings as a whole, and as are typically understood by those skilled in the art. In the event that a conflict arises between an art-understood definition of a word or phrase and a definition of the word or phrase as specifically taught in this specification, the specification shall control. Headings used herein are merely for convenience, and are not to be construed as limiting in any way.

As used herein, "DNA" refers to deoxyribonucleic acid in its various forms as understood in the art, such as genomic DNA, cDNA, isolated nucleic acid molecules, vector DNA, and chromosomal DNA. "Nucleic acid" refers to DNA or RNA (ribonucleic acid) in any form. As used herein, the term "isolated nucleic acid molecule" refers to a nucleic acid molecule (DNA or RNA) that has been removed from its native environment. Some examples of isolated nucleic acid molecules are recombinant DNA molecules contained in a vector, recombinant DNA molecules maintained in a heterologous host cell, partially or substantially purified nucleic acid molecules, and synthetic DNA molecules. An "isolated" nucleic acid can be free of sequences which naturally flank the nucleic acid (i.e., sequences located at the 5' and 3' ends of the nucleic acid) in the genomic DNA of the organism from which the nucleic acid is derived. Moreover, an "isolated" nucleic acid molecule, such as a cDNA molecule, can be substantially free of other cellular material or culture medium when produced by recombinant techniques, or of chemical precursors or other chemicals when chemically synthesized.

"Short tandem repeat" or "STR" loci refer to regions of genomic DNA which contain short, repetitive sequence elements. The sequence elements that are repeated are not limited to but are generally three to seven base pairs in length. Each sequence element is repeated at least once within an STR and is referred to herein as a "repeat unit." The term STR also encompasses a region of genomic DNA wherein more than a single repeat unit is repeated in tandem or with intervening bases, provided that at least one of the sequences is repeated at least two times in tandem.

"Polymorphic short tandem repeat loci" refers to STR loci in which the number of repetitive sequence elements (and net length of the sequence) in a particular region of genomic DNA varies from allele to allele, and from individual to individual.

As used herein, "allelic ladder" refers to a standard size marker consisting of amplified alleles from the locus. "Allele" refers to a genetic variation associated with a segment of DNA; i.e., one of two or more alternate forms of a DNA sequence occupying the same locus.

"Biochemical nomenclature" refers to the standard biochemical nomenclature as used herein, in which the nucleotide bases are designated as adenine (A), thymine (T), guanine (G), and cytosine (C). Corresponding nucleotides are, for example, deoxyguanosine-5'-triphosphate (dGTP).

"DNA polymorphism" refers to the condition in which two or more different nucleotide sequences in a DNA sequence coexist in the same interbreeding population.

"Locus" or "genetic locus" refers to a specific physical position on a chromosome. Alleles of a locus are located at identical sites on homologous chromosomes.

"Locus-specific primer" refers to a primer that specifically hybridizes with a portion of the stated locus or its complementary strand, at least for one allele of the locus, and does not hybridize efficiently with other DNA sequences under the conditions used in the amplification method.

"Polymerase chain reaction" or "PCR" refers to a technique in which repetitive cycles of denaturation, annealing with a primer, and extension with a DNA polymerase enzyme are used to amplify the number of copies of a target DNA sequence by approximately $10^6$ times or more. The PCR process for amplifying nucleic acids is covered by U.S. Patent Nos. 4,683,195 and 4,683,202, which are herein incorporated in their entirety by reference for a description of the process. The reaction conditions for any PCR comprise the chemical components of the reaction and their concentrations, the temperatures used in the reaction cycles, the number of cycles of the reaction, and the durations of the stages of the reaction cycles.

As used herein, "amplify" refers to the process of enzymatically increasing the amount of a specific nucleotide sequence. This amplification is not limited to but is generally accomplished by PCR. As used herein, "denaturation" refers to the separation of two complementary nucleotide strands from an annealed state. Denaturation can be induced by a number of factors, such as, for example, ionic strength of the buffer, temperature, or chemicals that disrupt base pairing interactions. As used herein, "annealing" refers to the specific interaction between strands of nucleotides wherein the strands bind to one another substantially based on complementarity between the strands as determined by Watson-Crick base pairing. It is not necessary that complementarity be 100% for annealing to occur. As used herein, "extension" refers to the amplification cycle after the primer oligonucleotide and target nucleic acid have annealed, wherein the polymerase enzyme effects primer extension into the appropriately sized fragments using the target nucleic acid as replicative template.

"Primer" refers to a single-stranded oligonucleotide or DNA fragment which hybridizes with a DNA strand of a locus in such a manner that the 3' terminus of the primer can act as a site of polymerization and extension using a DNA polymerase enzyme. "Primer pair" refers to two primers comprising a primer 1 that hybridizes to a single strand at one end of the DNA sequence to be amplified, and a primer 2 that hybridizes with the other end on the complementary strand of the DNA sequence to be amplified. "Primer site" refers to the area of the target DNA to which a primer hybridizes.

"Genetic markers" are generally alleles of genomic DNA with characteristics of interest for analysis, such as DNA typing, in which individuals are differentiated based on variations in their DNA. Most DNA typing methods are designed to detect and analyze differences in the length and/or sequence of one or more regions of DNA markers known to appear in at least two different forms, or alleles, in a population. Such variation is referred to as "polymorphism," and any region of DNA in which such a variation occurs is referred to as a "polymorphic locus." One possible method of performing DNA typing involves the joining of PCR amplification technology (KB Mullis, U.S. Patent No. 4,683,202) with the analysis of length variation polymorphisms. PCR traditionally could only be used to amplify relatively small DNA segments reliably; i.e., only amplifying DNA segments under 3,000 bases in length (M. Ponce and L. Micol (1992), NAR 20(3):623; R. Decorte et al. (1990), DNA CELL BIOL. 9(6):461 469). Short tandem repeats (STRs), minisatellites and variable number of tandem repeats (VNTRs) are some examples of length variation polymorphisms. DNA segments containing minisatellites or VNTRs are generally too long to be amplified reliably by PCR. By contrast STRs, containing repeat units of approximately three to seven nucleotides, are short enough to be useful as genetic markers in PCR applications, because amplification protocols can be designed to produce smaller products than are possible from the other variable length regions of DNA.

It is often desirable to amplify and detect multiple loci simultaneously in a single amplification reaction and separation process. Such systems simultaneously targeting several loci for analysis are called "multiplex" systems. Several such systems containing multiple STR loci have been described. *See, e.g.,* AMPFLSTR® SGMPLUS™ PCR AMPLIFICATION KIT USER'S MANUAL, Applied Biosystems, pp. i-x and 1-1 to 1-16 (2001); AMPFLSTR® IDENTIFILER® PCR AMPLIFICATION KIT USER'S MANUAL, Applied Biosystems, pp. i-x and 1-1 to 1-10 (2001); JW Schumm et al., U.S. Patent No. 7,008,771.

The governments of several countries maintain databases of DNA typing information. The National DNA Database of the United Kingdom (NDNAD) is the largest such database, with the DNA profiles of approximately 2.7 million people. H. Wallace (2006), EMBO REPORTS 7:S26-S30 (*citing* Home Office, 2006). Since 1999, the DNA profiles in the NDNAD have been based on the SGMplus® system, developed by Applied Biosystems. *Id.* A recurring problem in DNA profiling systems is how to identify individuals when their DNA samples are degraded. A number of studies have been performed in labs in Europe and the United States to compare conventional STRs (amplicons which range in size from about 100 to about 450 base pairs) with mini-STRs (amplicons of 200 base pairs or fewer) as genetic markers in analyzing degraded DNA samples. *See, e.g.,* LA Dixon et al. (2006), FORENSIC SCI. INT. 164(1):33-44. The results indicate that the chances of obtaining successful results from the analysis of degraded DNA samples improves with smaller sized amplicons, such as are obtained from mini-STR loci. *Id*.; MD Coble and JM Butler (2005), J. FORENSIC SCI. 50(1):43-53. The European Network of Forensic Science Institutes (ENFSI) and European DNA Profiling (EDNAP) group agreed that multiplex PCR systems for DNA typing should be re-engineered to enable small amplicon detection, and that standardization of profiling systems within Europe should take account of mini-STRs. P. Gill et al. (2006), FORENSIC SCI. INT. 156(2-3):242-244. The present teachings relate to the simultaneous analysis of multiple length variation polymorphisms in a single reaction. Various embodiments of the present teachings incorporate mini-STR loci in multiplex amplification systems. These systems are amenable to various applications, including their use in DNA typing.

The methods of the present teachings contemplate selecting an appropriate set of loci, primers, and amplification protocols to generate amplified alleles (amplicons) from multiple co-amplified loci, which amplicons can be designed so as not to overlap in size, and/or can be labeled in such a way as to enable one to differentiate between alleles from different loci which do overlap in size. In addition, these methods contemplate the selection of multiple STR loci which are compatible for use with a single amplification protocol. The specific combinations of loci described herein are unique in this application. In various embodiments of the present teachings a co-amplification of fifteen STR loci is taught, which comprises at least eight mini-STR loci with a maximum amplicon size of less than approximately 200 base pairs.

Successful combinations in addition to those disclosed herein can be generated by, for example, trial and error of locus combinations, by selection of primer pair sequences, and by adjustment of primer concentrations to identify an equilibrium in which all loci for analysis can be amplified. Once the methods and materials of these teachings are disclosed, various methods of selecting loci, primer pairs, and amplification techniques for use in the methods and kits of these teachings are likely to be suggested to one skilled in the art. All such methods are intended to be within the scope of the appended claims.

Practice of the methods of the present teaching may begin with selection of a set of at least eleven STR loci comprising D16S539, D18S51, D19S433, D21S11, D2S1338, D3S1358, D8S1179, FGA, TH01, V WA, and at least one of D10S1248, D12S391, D1S1656, D22S1045, and D2S441, all of which can be co-amplified in a single multiplex amplification reaction. Other loci besides or in addition to these 15 listed loci may be included in the multiplex amplification reaction. Possible methods for selecting the loci and oligonucleotide primers to amplify the loci in the multiplex amplification reaction of the present teachings are described herein and illustrated in the Example below.

Any of a number of different techniques can be used to select the set of loci for use according to the present teachings. One technique for developing useful sets of loci for use in this method of analysis is described below in the Example. Once a multiplex containing the at least eleven STR loci is developed, it can be used as a core to create multiplexes containing more than these eleven loci, and containing loci other than STR loci; for example, a sex determination locus. New combinations of more than eleven loci can thus be created comprising the first eleven STR loci.

Regardless of what methods may be used to select the loci analyzed by the methods of the present teaching, the loci selected for multiplex analysis in various embodiments share one or more of the following characteristics: (1) they produce sufficient amplification products to allow allelic evaluation of the DNA; (2) they generate few, if any, artifacts during the multiplex amplification step due to incorporation of additional bases during the extension of a valid target locus or the production of non-specific amplicons; and (3) they generate few, if any, artifacts due to premature termination of amplification reactions by a polymerase. *See, e.g.,* JW Schumm et al. (1993), FOURTH INTERNATIONAL SYMPOSIUM ON HUMAN IDENTIFICATION, pp. 177-187, Promega Corp.

The terms for the particular STR loci as used herein refer to the names assigned to these loci as they are known in the art. The loci are identified, for example, in the various references and by the various accession numbers in the list that follows, all of which are incorporated herein by reference in their entirety. The list of references that follows is merely intended to be exemplary of sources of locus information. The information regarding the DNA regions comprising these loci and contemplated for target amplification are publicly available and easily found by consulting the following or other references and/or accession numbers. Where appropriate, the current Accession Number as of time of filing is presented, as provided by GenBank® (National Center for Biotechnology Information, Bethesda, MD). *See, e.g.,* for the locus D3S1358, H. Li et al. (1993), HUM. MOL. GENET. 2:1327; for D12S391, MV Lareu et al. (1996), GENE 182:151-153; for D18S51, RE Staub et al. (1993), GENOMICS 15:48-56; for D21S11, V. Sharma and M. Litt (1992), Hum. MOL. GENET. 1:67; for FGA (FIBRA), KA Mills et al. (1992), HUM. MOL. GENET. 1:779; for TH01, A. Edwards (1991), AM. J. HUM. GENET. 49:746-756 and MH Polymeropoulos et al. (1991), NUCLEIC ACIDS RES. 19:3753; for VWA (vWF), CP Kimpton et al. (1992), HUM. MOL. GENET. 1:287; for D10S1248, MD Coble and JM Butler (2005), J. FORENSIC SCI. 50(1):43-53; for D16S539, J. Murray et al. (1995), unpublished, Cooperative Human Linkage Center, Accession Number G07925; for D2S 1338, J. Murray et al. (1995), unpublished, Cooperative Human Linkage Center, Accession Number G08202 and Watson et al. in PROGRESS IN FORENSIC GENETICS 7: PROCEEDINGS OF THE 17TH INT'L ISFH CONGRESS, OSLO, 2-6 SEPTEMBER 1997, B. Olaisen et al., eds., pp. 192-194 (Elsevier, Amsterdam); for D8S1179, J. Murray et al. (1995), unpublished, Cooperative Human Linkage Center, Accession Number G08710, and NJ Oldroyd et al. (1995), ELECTROPHORESIS 16:334-337; for D22S1045, J. Murray et al. (1995), unpublished, Cooperative Human Linkage Center, Accession Number G08085; for D19S433, J. Murray et al. (1995), unpublished, Cooperative Human Linkage Center, Accession Number G08036, and MV Lareu et al. (1997), in PROGRESS IN FORENSIC GENETICS 7: PROCEEDINGS OF THE 17TH INT'L ISFH CONGRESS, OSLO, 2-6 SEPTEMBER 1997, B. Olaisen et al., eds., pp. 192-200, Elsevier, Amsterdam; for D2S441, J. Murray et al. (1995), unpublished, Cooperative Human Linkage Center, Accession Number G08184; for D1S1656, J. Murray et al. (1995), unpubl ished, Cooperative Human Linkage Center, Accession Number G07820.

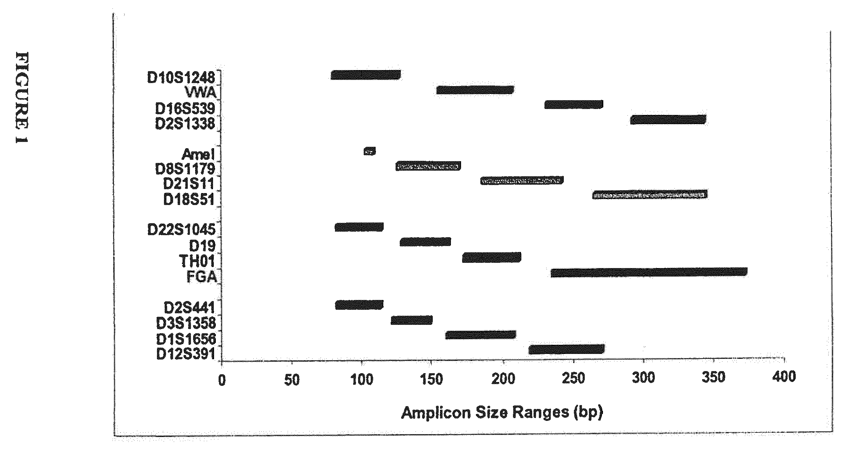

Amplification of mini-STRs (loci of fewer than approximately 200 base pairs) allows for the profiling analysis of highly degraded DNA, as is demonstrated in MD Coble (2005), J. FORENSIC SCI. 50(1):43-53, which is incorporated by reference herein. Figure 1 demonstrates the locus size ranges for all fifteen loci described above, plus the Amelogenin locus for size determination. As can be seen in Figure 1, eight of the loci identified in the preceding list comprise such mini-STR loci: D10S1248, VWA, D8S1179, D22S1045, D19S433, D2S441, D3S 1358 and D1S1656.

The set of loci selected for co-amplification and analysis according to these teachings can comprise at least one locus in addition to the at least eleven STR loci. The additional locus can comprise an STR or other sequence polymorphism, or any other feature, for example, which identifies a particular characteristic to separate the DNA of one individual from the DNA of other individuals in the population. The additional locus can also be one which identifies the sex of the source of the DNA sample analyzed. When the DNA sample is human genomic DNA, a sex-identifying locus such as the Amelogenin locus can be selected for co-amplification and analysis according to the present methods. The Amelogenin locus is identified by GenBank as HUMAMELY (when used to identify a locus on the Y chromosome as present in male DNA) or as HUMAMELX (when used to identify a locus on the X chromosome as present in male or female DNA).

Once a set of loci for co-amplification in a single multiplex reaction is identified, one can determine primers suitable for co-amplifying each locus in the set. Oligonucleotide primers may be added to the reaction mix and serve to demarcate the 5' and 3' ends of an amplified DNA fragment. One oligonucleotide primer anneals to the sense (+) strand of the denatured template DNA, and the other oligonucleotide primer anneals to the antisense (-) strand of the denatured template DNA. Typically, oligonucleotide primers may be approximately 12-25 nucleotides in length, but their size may vary considerably depending on such parameters as, for example, the base composition of the template sequence to be amplified, amplification reaction conditions, etc. The specific length of the primer is not essential to the operation of these teachings. Oligonucleotide primers can be designed to anneal to specific portions of DNA that flank a locus of interest, so as to specifically amplify the portion of DNA between the primer-complementary sites.

Oligonucleotide primers may comprise adenosine, thymidine, guanosine, and cytidine, as well as uracil, nucleoside analogs (for example, but not limited to, inosine, locked nucleic acids (LNA), non-nucleotide linkers, peptide nucleic acids (PNA) and phosporamidites) and nucleosides containing or conjugated to chemical moieties such as radionuclides (e.g., $^{32}$P and $^{35}$S), fluorescent molecules, minor groove binders (MGBs), or any other nucleoside conjugates known in the art.

Generally, oligonucleotide primers can be chemically synthesized. Primer design and selection is a routine procedure in PCR optimization. One of ordinary skill in the art can easily design specific primers to amplify a target locus of interest, or obtain primer sets from the references listed herein. All of these primers are within the scope of the present teachings.

Care should be taken in selecting the primer sequences used in the multiplex reaction. Inappropriate selection of primers may produce undesirable effects such as a lack of amplification, amplification at one site or multiple sites besides the intended target locus, primer-dimer formation, undesirable interactions between primers for different loci, production of amplicons from alleles of one locus which overlap (e.g., in size) with alleles from another locus, or the need for amplification conditions or protocols particularly suited for each of the different loci, which conditions/protocols are incompatible in a single multiplex system. Primers can be developed and selected for use in the multiplex systems of this teaching by, for example, employing a re-iterative process of multiplex optimization that is well familiar to one of ordinary skill in the art: selecting primer sequences, mixing the primers for co-amplification of the selected loci, co-amplifying the loci, then separating and detecting the amplified products to determine effectiveness of the primers in amplification.

As an example of primer selection, individual primers and primer pairs, identified in the references cited herein or described in other references, which are useful in amplifying any of the above listed loci may be selected to amplify and analyze the STR loci according to the present teachings. As another example, primers can be selected by the use of any of various software programs available and known in the art for developing amplification and/or multiplex systems. *See, e.g.,* Primer Express® software (Applied Biosystems, Foster City, Calif.). In the example of the use of software programs, sequence information from the region of the locus of interest can be imported into the software. The software then uses various algorithms to select primers that best meet the user's specifications.

Initially, this primer selection process may produce any of the undesirable effects in amplification described above, or an imbalance of amplification product, with greater product yield for some loci than for others because of greater binding strength between some primers and their respective targets than other primers, for example resulting in preferred annealing and amplification for some loci. Or, the primers may generate amplification products which do not represent the target loci alleles themselves; i.e., non-specific amplification product may be generated. These extraneous products resulting from poor primer design may be due, for example, to annealing of the primer with non-target regions of sample DNA, or even with other primers, followed by amplification subsequent to annealing.

When imbalanced or non-specific amplification products are present in the multiplex systems during primer selection, individual primers can be taken from the total multiplex set and used in an amplification with primers from the same or other loci to identify which primers contribute to the amplification imbalance or artifacts. Once two primers which generate one or more of the artifacts or imbalance are identified, one or both contributors can be modified and retested, either alone in a pair, or in the multiplex system (or a subset of the multiplex system). This process may be repeated until product evaluation results in amplified alleles with no or an acceptable level of amplification artifacts in the multiplex system.

The optimization of primer concentration can be performed either before or after determination of the final primer sequences, but most often may be performed after primer selection. Generally, increasing the concentration of primers for any particular locus increases the amount of product generated for that locus. However, primer concentration optimization is also a re-iterative process because, for example, increasing product yield from one locus may decrease the yield from another locus or other loci. Furthermore, primers may interact with each other, which may directly affect the yield of amplification product from various loci. In sum, a linear increase in concentration of a specific primer set does not necessarily equate with a linear increase in amplification product yield for the corresponding locus. Reference is made to MJ Simons, U.S. Patent No. 5,192,659, for a more detailed description of locus-specific primers, the teaching of which is incorporated herein by reference in its entirety.

Locus-to-locus amplification product balance in a multiplex reaction may also be affected by a number of parameters of the amplification protocol, such as, for example, the amount of template (sample DNA) input, the number of amplification cycles used, the annealing temperature of the thermal cycling protocol, and the inclusion or exclusion of an extra extension step at the end of the cycling process. An absolutely even balance in amplification product yield across all alleles and loci, although theoretically desirable, is generally not achieved in practice.

The process of determining the loci comprising the multiplex system and the development of the reaction conditions of this system can also be a re-iterative process. That is, one can first develop a multiplex system for a small number of loci, this system being free or nearly free of amplification artifacts and product imbalance. Primers of this system can then be combined with primers for another locus or several additional loci desired for analysis. This expanded primer combination may or may not produce amplification artifacts or imbalanced product yield. In turn, some loci may be removed from the system, and/or new loci can be introduced and evaluated.

One or more of the re-iterative selection processes described above can be repeated until a complete set of primers is identified, which can be used to co-amplify the at least eleven loci selected for co-amplification as described above, comprising the STR loci VWA, D16S539, D2S1338, D8S1179, D21S11, D18S51, D19S433, TH01, FGA, D3S1358, and one or more of D10S1248, D22S1045, D2S441, D1S1656, and D12S391. It is understood that many different sets of primers can be developed to amplify a particular set of loci. Synthesis of the primers used in the present teachings can be conducted using any standard procedure for oligonucleotide synthesis known to those skilled in the art and/or commercially available. In various embodiments of the present teaching, all fifteen of these STR loci can be co-amplified in one multiplex amplification system: VWA, D16S539, D2S1338, D8S1179, D21S11, D18S51, D195433, TH01, FGA, D3S1358, D10S1248, D22S1045, D2S441, D1S1656, and D12S391. In other embodiments of the present teaching, all fifteen of these STR loci can be co-amplified in one multiplex amplification system, as well as and including the Amelogenin locus for sex determination of the source of the DNA sample.

Samples of genomic DNA can be prepared for use in the methods of the present teaching using any procedures for sample preparation that are compatible with the subsequent amplification of DNA. Many such procedures are known by those skilled in the art. Some examples are DNA purification by phenol extraction (J. Sambrook et al. (1989), in MOLECULAR CLONING: A LABORATORY MANUAL, SECOND EDITION, Cold Spring Harbor Laboratory Press, Cold Spring Harbor, N.Y., pp. 9.14-9.19), and partial purification by salt precipitation (S. Miller et al. (1988), NUCL. ACIDS RES. 16:1215) or chelex (PS Walsh et al. (1991), BIOTECHNIQUES 10:506-513; CT Comey et al. (1994), J. FORENSIC SCI. 39:1254) and the release of unpurified material using untreated blood (J. Burckhardt (1994), PCR METHODS AND APPLICATIONS 3:239-243; RBE McCabe (1991), PCR METHODS AND APPLICATIONS 1:99-106; BY Nordvag (1992), BIOTECHNIQUES 12:4 pp. 490-492).

When the at least one DNA sample to be analyzed using the methods of this teaching is human genomic DNA, the DNA can be prepared from tissue samples such as, for example, one or more of blood, semen, vaginal cells, hair, saliva, urine, bone, buccal samples, amniotic fluid containing placental cells or fetal cells, chorionic villus, and/or mixtures of any of these or other tissues.

Optionally, DNA concentrations can be measured prior to use in the method of the present teaching, using any standard method of DNA quantification known to those skilled in the art. Such quantification methods include, for example, spectrophotometric measurement, as described by J. Sambrook et al. (1989), *supra,* Appendix E.5; or fluorometric methodology using a measurement technique such as that described by CF Brunk et al. (1979), ANAL. BIOCHEM. 92: 497-500. DNA concentration can be measured by comparison of the amount of hybridization of DNA standards with a human-specific probe such as that described by JS Waye et al. (1991), J. FORENSIC SCI. 36:1198-1203 (1991). Use of too much template DNA in the amplification reactions may produce amplification artifacts, which would not represent true alleles.

Once a sample of genomic DNA is prepared, the target loci can be co-amplified in the multiplex amplification step of the present teaching. Any of a number of different amplification methods can be used to amplify the loci, such as, for example, PCR (RK Saiki et al. (1985), SCIENCE 230: 1350-1354), transcription based amplification (DY Kwoh and TJ Kwoh (1990), AMERICAN BIOTECHNOLOGY LABORATORY, October, 1990) and strand displacement amplification (SDA) (GT Walker et al. (1992), PROC. NATL. ACAD. SCI., U.S.A. 89: 392-396). In some embodiments of the present teaching, multiplex amplification can be effected via PCR, in which the DNA sample is subjected to amplification using primer pairs specific to each locus in the multiplex.

The chemical components of a standard PCR generally comprise a solvent, DNA polymerase, deoxyribonucleoside triphosphates ("dNTPs"), oligonucleotide primers, a divalent metal ion, and a DNA sample expected to contain the target(s) for PCR amplification. Water can generally be used as the solvent for PCR, typically comprising a buffering agent and non-buffering salts such as KCl. The buffering agent can be any buffer known in the art, such as, but not limited to, Tris-HCl, and can be varied by routine experimentation to optimize PCR results. Persons of ordinary skill in the art are readily able to determine optimal buffering conditions. PCR buffers can be optimized depending on the particular enzyme used for amplification.

Divalent metal ions can often be advantageous to allow the polymerase to function efficiently. For example, the magnesium ion is one which allows certain DNA polymerases to function effectively. Typically $MgCl_2$ or $MgSO_4$ can be added to reaction buffers to supply the optimum magnesium ion concentration. The magnesium ion concentration required for optimal PCR amplification may depend on the specific set of primers and template used. Thus, the amount of magnesium salt added to achieve optimal amplification is often determined empirically, and is a routine practice in the art. Generally, the concentration of magnesium ion for optimal PCR can vary between about 1 and about 10 mM. A typical range of magnesium ion concentration in PCR can be between about 1.0 and about 4.0 mM, varying around a midpoint of about 2.5 mM. Alternatively, the divalent ion manganese can be used, for example in the form of manganese dioxide ($MnO_2$), titrated to a concentration appropriate for optimal polymerase activity, easily determined by one of skill in the art using standard laboratory procedures.

The dNTPs, which are the building blocks used in amplifying nucleic acid molecules, can typically be supplied in standard PCR at a concentration of, for example, about 40-200 $\mu$M each of deoxyadenosine triphosphate ("dATP"), deoxyguanosine triphosphate ("dGTP"), deoxycytidine triphosphate ("dCTP") and deoxythymidine triphosphate ("dTTP"). Other dNTPs, such as deoxyuridine triphosphate ("dUTP"), dNTP analogs (e.g., inosine), and conjugated dNTPs can also be used, and are encompassed by the term "dNTPs" as used herein. While use of dNTPs at concentrations of about 40-200 $\mu$M each can be amenable to the methods of this teaching, concentrations of dNTPs higher than about 200 $\mu$M each could be advantageous. Thus, in some embodiments of the methods of these teachings, the concentration of each dNTP is generally at least about 500 $\mu$M and can be up to about 2 mM. In some further embodiments, the concentration of each dNTP may range from about 0.5 mM to about 1 mM. Specific dNTP concentrations used for any multiplex amplification can vary depending on multiplex conditions, and can be determined empirically by one of skill in the art using standard laboratory procedures.

The enzyme that polymerizes the nucleotide triphosphates into the amplified products in PCR can be any DNA polymerase. The DNA polymerase can be, for example, any heat-resistant polymerase known in the art. Examples of some polymerases that can be used in this teaching are DNA polymerases from organisms such as *Thermus aquaticus, Thermus thermophilus, Thermococcus litoralis, Bacillus stearothermophilus, Thermotoga maritima* and *Pyrococcus sp.* The enzyme can be acquired by any of several possible methods; for example, isolated from the source bacteria, produced by recombinant DNA technology or purchased from commercial sources. Some examples of such commercially available DNA polymerases include AmpliTaq Gold® DNA polymerase; AmpliTaq® DNA Polymerase; AmpliTaq® DNA Polymerase Stoffel Fragment; rTth DNA Polymerase; and rTth DNA Polymerase, XL (all manufactured by Applied Biosystems, Foster City, Calif.) Other examples of suitable polymerases include Tne, Bst DNA polymerase large fragment from *Bacillus stearothermophilus,* Vent and Vent Exo- from *Thermococcus litoralis,* Tma from *Thermotoga maritima,* Deep Vent and Deep Vent Exo- and Pfu from *Pyrococcus sp.,* and mutants, variants and derivatives of the foregoing.

Other known components of PCR can be used within the scope of the present teachings. Some examples of such components include sorbitol, detergents (e.g., Triton X-100, Nonidet P-40 (NP-40), Tween-20) and agents that disrupt mismatching of nucleotide pairs, such as, for example, dimethylsulfoxide (DMSO), and tetramethylammonium chloride (TMAC), and uracil N-glycosylase or other agents which act to prevent amplicon contamination of the PCR and/or unwanted generation of product during incubation or preparation of the PCR, before the PCR procedure begins.

PCR cycle temperatures, the number of cycles and their durations can be varied to optimize a particular reaction, as a matter of routine experimentation. Those of ordinary skill in the art will recognize the following as guidance in determining the various parameters for PCR, and will also recognize that variation of one or more conditions is within the scope of the present teachings. Temperatures and cycle times are determined for three stages in PCR: denaturation, annealing and extension. One round of denaturation, annealing and extension is referred to as a "cycle." Denaturation can generally be conducted at a temperature high enough to permit the strands of DNA to separate, yet not so high as to destroy polymerase activity. Generally, thermoresistant polymerases can be used in the reaction, which do not denature but retain some level of activity at elevated temperatures. However, heat-labile polymerases can be used if they are replenished after each denaturation step of the PCR. Typically, denaturation can be conducted above about 90° C and below about 100° C. In some embodiments, denaturation can be conducted at a temperature of about 94-95° C. Denaturation of DNA can generally be conducted for at least about 1 to about 30 seconds. In some embodiments, denaturation can be conducted for about 1 to about 15 seconds. In other embodiments, denaturation can be conducted for up to about 1 minute or more. In addition to the denaturation of DNA, for some polymerases, such as AmpliTaq Gold®, incubation at the denaturation temperature also can serve to activate the enzyme. Therefore, it can be advantageous to allow the first denaturation step of the PCR to be longer than subsequent denaturation steps when these polymerases are used.

During the annealing phase, oligonucleotide primers anneal to the target DNA in their regions of complementarity and are substantially extended by the DNA polymerase, once the latter has bound to the primer-template duplex. In a conventional PCR, the annealing temperature can typically be at or below the melting point ($T_m$) of the least stable primer-template duplex, where $T_m$ can be estimated by any of several theoretical methods well known to practitioners of the art. For example, $T_m$ can be determined by the formula:

$$T_m = (4°C \times \text{number of G and C bases}) + (2°C \times \text{number of A and T bases}).$$

Typically, in standard PCR, the annealing temperature can be about 5-10°C below the estimated $T_m$ of the least stable primer-template duplex. The annealing time can be between about 30 seconds and about 2 minutes. The annealing phase is typically followed by an extension phase. Extension can be conducted for a sufficient amount of time to allow the polymerase enzyme to complete primer extension into the appropriately sized amplification products.

The number of cycles in the PCR (one cycle comprising denaturation, annealing and extension) determines the extent of amplification and the subsequent amount of amplification product. PCR results in an exponential amplification of DNA molecules. Thus, theoretically, after each cycle of PCR there are twice the number of products that were present in the previous cycle, until PCR reagents are exhausted and a plateau is reached at which no further amplification products are generated. Typically, about 20-30 cycles of PCR may be performed to reach this plateau. More typically, about 25-30 cycles may be performed, although cycle number is not particularly limited.

For some embodiments, it can be advantageous to incubate the reactions at a certain temperature following the last phase of the last cycle of PCR. In some embodiments, a prolonged extension phase can be selected. In other embodiments, an incubation at a low temperature (e.g., about 4°C) can be selected.

Various methods can be used to evaluate the products of the amplified alleles in the mixture of amplification products obtained from the multiplex reaction including, for example, detection of fluorescent labeled products, detection of radioisotope labeled products, silver staining of the amplification products, or the use of DNA intercalator dyes such as ethidium bromide (EtBr) and SYBR green cyanine dye to visualize double-stranded amplification products. Fluorescent labels suitable for attachment to primers for use in the present teachings are numerous, commercially available, and well-known in the art. With fluorescent analysis, at least one fluorescent labeled primer can be used for the amplification of each locus. Fluorescent detection may be desirable over radioactive methods of labeling and product detection, for example, because fluorescent detection does not require the use of radioactive materials, and thus avoids the regulatory and safety problems that accompany the use of radioactive materials. Fluorescent detection with labeled primers may also be selected over other non-radioactive methods of detection, such as silver staining and DNA intercalators, because fluorescent methods of detection generally reveal fewer amplification artifacts than do silver staining and DNA intercalators. This is due in part to the fact that only the amplified strands of DNA with labels attached thereto are detected in fluorescent detection, whereas both strands of every amplified product are stained and detected using the silver staining and intercalator methods of detection, which result in visualization of many non-specific amplification artifacts. Additionally, there are potential health risks associated with the use of EtBr and SYBR. EtBr is a known mutagen; SYBR, although less of a mutagen than EtBr, is generally suspended in DMSO, which can rapidly pass through skin.

Where fluorescent labeling of primers is used in a multiplex reaction, generally at least three different labels can be used to label the different primers. When a size marker is used to evaluate the products of the multiplex reaction, the primers used to prepare the size marker may be labeled with a different label from the primers that amplify the loci of interest in the reaction. With the advent of automated fluorescent imaging and analysis, faster detection and analysis of multiplex amplification products can be achieved.

In some embodiments of the present teaching, a fluorophore can be used to label at least one primer of the multiplex amplification, e.g. by being covalently bound to the primer, thus creating a fluorescent labeled primer. In some embodiments, primers for different target loci in a multiplex can be labeled with different fluorophores, each fluorophore producing a different colored product depending on the emission wavelength of the fluorophore. These variously labeled primers can be used in the same multiplex reaction, and their respective amplification products subsequently analyzed together. Either the forward or reverse primer of the pair that amplifies a specific locus can be labeled, although the forward may more often be labeled.

The following are some examples of possible fluorophores well known in the art and suitable for use in the present teachings. The list is intended to be exemplary and is by no means exhaustive. Some possible fluorophores include: fluorescein (FL), which absorbs maximally at 492 nm and emits maximally at 520 nm; N,N,N',N'-tetramethyl-6-carboxyrhodamine (TAMRA™), which absorbs maximally at 555 nm and emits maximally at 580 nm; 5-carboxyfluorescein (5-FAM™), which absorbs maximally at 495 nm and emits maximally at 525 nm; 2',7'-dimethoxy-4',5'-dichloro-6-carboxyfluorescein (JOE™), which absorbs maximally at 525 nm and emits maximally at 555 nm); 6-carboxy-X-rhodamine (ROX™), which absorbs maximally at 585 nm and emits maximally at 605 nm; CY3 ™, which absorbs maximally at 552 nm and emits maximally at 570 nm; CY5™, which absorbs maximally at 643 nm and emits maximally at 667 nm; tetrachloro-fluorescein (TET™), which absorbs maximally at 521 nm and emits maximally at 536 nm; and hexachloro-fluorescein (HEX™), which absorbs maximally at 535 nm and emits maximally at 556 nm; NED™, which absorbs maximally at 546 nm and emits maximally at 575 nm; 6-FAM™, which emits maximally at approximately 520 nm; VIC® which emits maximally at approximately 550 nm; PET® which emits maximally at approximately 590 nm; and LIZ™, which emits maximally at approximately 650 nm. *See* SR Coticone et al., U.S. Patent No. 6,780,588; AMPFLSTR® IDENTIFILER™ PCR AMPLIFICATION KIT USER'S MANUAL, pp. 1-3, Applied Biosystems (2001). Note that the above listed emission and/or absorption wavelengths are typical and can be used for general guidance purposes only; actual peak wavelengths may vary for different applications and under different conditions.

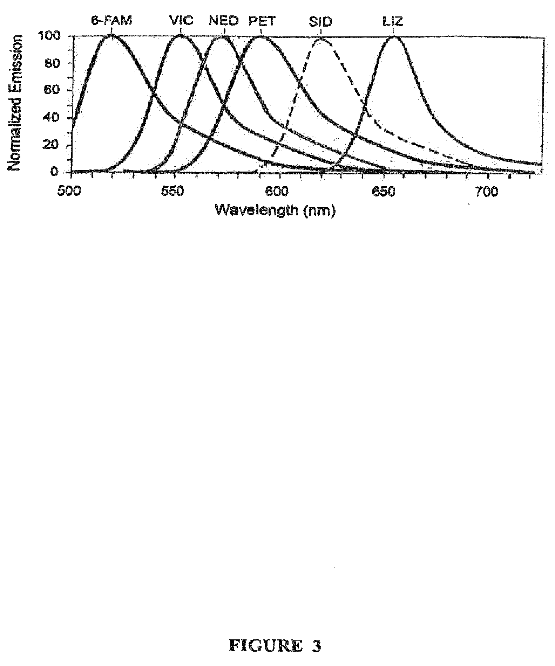

Various embodiments of the present teachings may comprise a single multiplex system comprising at least four different dyes. These at least four dyes may comprise any four of the above-listed dyes, or any other four dyes known in the art, or 6-FAM™, VIC®, NED™ and PET®. Other embodiments of the present teaching may comprise a single multiplex system comprising at least five different dyes. These at least five dyes may comprise any five of the above-listed dyes, or any other five dyes known in the art, or 6-FAM™, VIC®, NED™, PET® and LIZ™. See Figure 2 for an example of a DNA profile generated from the multiplex amplification of sixteen loci using the five dyes 6-FAM™, VIC®, NED™, PET® and LIZ™, as described in the Example (amplification peaks for LIZ™ not shown, as LIZ™ was used to label the size standards.) Other embodiments of the present teaching may comprise a single multiplex system comprising at least six different dyes. These at least six dyes may comprise any six of the above-listed dyes, or any other six dyes known in the art, or 6-FAM™, VIC®, NED™, PET®, LIZ™ and a sixth dye (SID) with maximum emission at approximately 620 nm. *See* Figure 3.

The PCR products can be analyzed on a sieving or non-sieving medium. In some embodiments of these teachings, for example, the PCR products can be analyzed by electrophoresis; e.g., capillary electrophoresis, as described in H. Wenz et al. (1998), GENOME RES. 8:69-80 *(see also* E. Buel et al. (1998), J. FORENSIC SCI. 43:( I), pp. 164-170)), or slab gel electrophoresis, as described in M. Christensen et al. (1999), SCAND. J. CLIN. LAB. INVEST. 59(3): 167-177, or denaturing polyacrylamide gel electrophoresis (*see, e.g.,* J. Sambrook et al. (1989), in MOLECULAR CLONING: A LABORATORY MANUAL, SECOND EDITION, Cold Spring Harbor Laboratory Press, Cold Spring Harbor, N.Y., pp. 13.45-13.57). The separation of DNA fragments in electrophoresis is based primarily on differential fragment size. Amplification products can also be analyzed by chromatography; e.g., by size exclusion chromatography (SEC).

Once the amplified alleles are separated, these alleles and any other DNA in, for example, the gel or capillary (e.g., a DNA size markers or an allelic ladder) can then be visualized and analyzed. Visualization of the DNA can be accomplished using any of a number of techniques known in the art, such as, for example, silver staining or by use of reporters such as radioisotopes and fluorescent dyes, as described herein, or chemiluminescers and enzymes in combination with detectable substrates. Oftentimes, the method for detection of multiplex loci can be by fluorescence. *See, e.g.,* JW Schumm et al. in PROCEEDINGS FROM THE EIGHTH INTERNATIONAL SYMPOSIUM ON HUMAN IDENTIFICATION, pub. 1998 by Promega Corporation, pp. 78-84; E. Buel et al. (1998), *supra.* Where fluorescent-labeled primers are used for detecting each locus in the multiplex reaction, amplification can be followed by detection of the labeled products employing a fluorometric detector. *See* the description of fluorescent dyes, *supra.*

The size of the alleles present at each locus in the DNA sample can be determined by comparison to a size standard in electrophoresis, such as a DNA marker of known size. Markers for evaluation of a multiplex amplification containing two or more polymorphic STR loci may also comprise a locus-specific allelic ladder or a combination of allelic ladders for each of the loci being evaluated. *See, e.g.,* C. Puers et al. (1993), AM. J. HUM. GENET. 53:953-958; C. Puers et al. (1994), GENOMICS 23:260-264. *See also,* U.S. Patent Nos. 5,599,666; 5,674,686; and 5,783,406 for descriptions of some allelic ladders suitable for use in the detection of STR loci, and some methods of ladder construction disclosed therein. Following the construction of allelic ladders for individual loci, the ladders can be electrophoresed at the same time as the amplification products. Each allelic ladder co-migrates with the alleles from the corresponding locus.

The products of the multiplex reactions of the present teachings can also be evaluated using an internal lane standard; i.e., a specialized type of size marker configured to be electrophoresed, for example, in the same capillary as the amplification products. The internal lane standard can comprise a series of fragments of known length. The internal lane standard can also be labeled with a fluorescent dye, which is distinguishable from other dyes in the amplification reaction. The lane standard can be mixed with amplified sample or size standards/allelic ladders and electrophoresed with either, in order to compare migration in different lanes of gel electrophoresis or different capillaries of capillary electrophoresis. Variation in the migration of the internal lane standard can serve to indicate variation in the performance of the separation medium. Quantitation of this difference and correlation with the allelic ladders can provide for calibration of amplification product electrophoresed in different lanes or capillaries, and correction in the size determination of alleles in unknown samples.

Where fluorescent dyes are used to label amplification products, the electrophoresed and separated products can be analyzed using fluorescence detection equipment such as, for example, the ABI PRISM® 310 or 3130xl genetic analyzer, or an ABI PRISM® 377 DNA Sequencer (Applied Biosystems, Foster City, Calif.); or a Hitachi FMBIO™ II Fluorescent Scanner (Hitachi Software Engineering America, Ltd., South San Francisco, Calif.). In various embodiments of the present teachings, PCR products can be analyzed by a capillary gel electrophoresis protocol in conjunction with such electrophoresis instrumentation as the ABI PRISM® 3130xl genetic analyzer (Applied Biosystems), and allelic analysis of the electrophoresed amplification products can be performed, for example, with GeneMapper®*ID* Software v3.2, from Applied Biosystems. In other embodiments, the amplification products can be separated by electrophoresis in, for example, about a 4.5%, 29:1 acrylamide:bis acrylamide, 8 M urea gel as prepared for an ABI PRISM® 377 Automated Fluorescence DNA Sequencer.

The present teachings are also directed to kits that utilize the processes described above. In some embodiments, a basic kit can comprise a container having one or more locus-specific primers. A kit can also optionally comprise instructions for use. A kit can also comprise other optional kit components, such as, for example, one or more of an allelic ladder directed to each of the specified loci, a sufficient quantity of enzyme for amplification, amplification buffer to facilitate the amplification, divalent cation solution to facilitate enzyme activity, dNTPs for strand extension during amplification, loading solution for preparation of the amplified material for electrophoresis, genomic DNA as a template control, a size marker to insure that materials migrate as anticipated in the separation medium, and a protocol and manual to educate the user and limit error in use. The amounts of the various reagents in the kits also can be varied depending upon a number of factors, such as the optimum sensitivity of the process. It is within the scope of these teachings to provide test kits for use in manual applications or test kits for use with automated detectors or analyzers.

Personal identification tests, or DNA typing, can be performed on any specimen that contains nucleic acid, such as bone, hair, blood, tissue and the like. DNA can be extracted from the specimen and a panel of primers used to amplify a desired set of STR loci of the DNA in a multiplex to generate a set of amplification products, as described herein. In forensic testing, the particular specimen's amplification pattern, or DNA profile, can be compared with a known sample taken from the presumptive victim (the presumed matching source), or can be compared to the pattern of amplified loci derived from the presumptive victim's family members (e.g., the mother and/or father) wherein the same set of STR loci is amplified. The pattern of STR loci amplification can be used to confirm or rule out the identity of the victim. In paternity testing, the test specimen generally can be from the child and comparison can be made to the STR loci pattern from the presumptive father, and/or can be matched with the STR loci pattern from the child's mother. The pattern of STR loci amplification can be used to confirm or rule out the identity of the father. The amplification and comparison of specific loci can also be used in paternity testing in a breeding context; e.g., for cattle, dogs, horses and other animals. CR Primmer et al. (1995), MOL. ECOL. 4:493-498.

In a clinical setting, such STR markers can be used, for example, to monitor the degree of donor engraftment in bone marrow transplants. In hospitals, these markers can also be useful in specimen matching and tracking. These markers have also entered other fields of science, such as population biology studies on human racial and ethnic group differences (DB Goldstein et al. (1995), PROC. NATL. ACAD. SCI, U.S.A. 92:6723-6727), evolution and species divergence, and variation in animal and plant taxa (MW Bruford et al. (1993), CURIO BIOL. 3:939-943).

The reference works, patents, patent applications, scientific literature and other printed publications, as well as accession numbers to GenBank database sequences that are referred to herein, are all hereby incorporated by reference in their entirety.

## EXAMPLE

Aspects of the present teachings can be further understood in light of the following example, which should not be construed as limiting the scope of the present teachings in any way.

In certain embodiments, a DNA sample to be analyzed was combined with STRand Amelogenin-specific primer sets in a PCR mixture to amplify the loci D16S539, D18S51, D19S433, D21S11, D2S 1338, D3S1358, D8S1179, FGA, TH01, VWA, Amelogenin, and five new STR loci D10S1248, D125391, D1S1656, D22S1045, and D2S441. Primer sets for these loci were designed according to the methodology provided herein, *supra.* One primer from each of the primer sets that amplify D10S1248, VWA, D16S539 and D2S1338 was labeled with the 6-FAM™ fluorescent label. One primer from each of the primer sets that amplify Amelogenin, D8S 1179, D21S11 and D18S51 was labeled with the VIC® fluorescent label. One primer from each of the primer sets that amplify D22S1045, D195433, TH01 and FGA was labeled with the NED™ fluorescent label. One primer from each of the primer sets that amplify D2S441, D3S1358, D1S1656 and D125391 was labeled with the PET® fluorescent label. A fifth fluorescent label, LIZ™, was used to label a size standard.

The reaction mixture was then subjected to polymerase chain reaction. Amplification products were generated from the STR and Amelogenin loci, with the labeled primers becoming incorporated into the amplification products. Amplification products were thus labeled with the 6-FAM™, VIC®, NED™ or PET® fluorescent labels. All or a portion of the reaction mixture was subjected to capillary electrophoresis following amplification, in a single capillary channel. The LIZ™-labeled size standard was also electrophoresed. Emission from the fluorescent labels was detected and displayed in a single output. See Figure 2 for the DNA profile from the amplification of the 16 loci using five dyes (LIZ™-labeled size standard not shown). The rate at which the STR and Amelogenin loci migrate through the channel is a function of their size. The size of the STR and Amelogenin amplification products and the color of their labels identified the alleles at each locus.

As those skilled in the art will appreciate, numerous changes and modifications may be made to the various embodiments of the present teachings without departing from the spirit of these teachings. It is intended that all such variations fall within the scope of these teachings.

Disclosed is:

1. A method comprising:

(a) co-amplifying a set of loci of at least one DNA sample to be analyzed in a multiplex amplification reaction, wherein the product of the reaction is a mixture of amplified alleles from the co-amplified loci in the set, wherein the set of loci comprises the 10 STR loci D16S539, D18S51, D19S433, D21S11, D2S1338, D3S1358, D8S1179, FGA, TH01, VWA; and one or more from the group consisting of the STR loci D10S1248, D12S391, D1S1656, D22S1045, and D2S441;

(b) evaluating the amplified alleles in the mixture to determine the alleles present at each of the loci analyzed in the set of loci within the at least one DNA sample.

2. The method of item 1, wherein the set of loci in step (a) further comprises a locus which can be used to identify the sex of the source(s) of the at least one DNA sample, preferably wherein the source of the DNA sample(s) is a human being, and the locus used to identify the sex of the human being is an Amelogenin locus.

3. The method of item 1, further comprising the step of separating the amplified alleles prior to the evaluating step, preferably wherein the amplified alleles are separated by capillary gel electrophoresis.

4. The method of item 1, wherein the co-amplifying step comprises using one pair of oligonucleotide primers for each of the loci in the set of loci, each of said pair of primers flanking a locus of the set of loci in the multiplex reaction.

5. The method of item 4, wherein at least one primer of each pair of oligonucleotide primers is a labeled primer, preferably said labeled primer is a fluorescent label.

6. The method of item 5, wherein the co-amplifying step comprises using at least five fluorescently labeled oligonucleotide primers, wherein the at least five labeled primers have at least five different fluorescent labels respectively covalently attached thereto.

7. The method of item 5, wherein the co-amplifying step comprises using at least six fluorescently labeled oligonucleotide primers, wherein the at least six labeled primers have at least six different fluorescent labels respectively covalently attached thereto, preferably wherein the at least six different fluorescent labels comprise a first fluorescent label which emits its maximum fluorescence at 520nm, a second fluorescent label which emits its maximum fluorescence at 550nm, a third fluorescent label which emits its maximum fluorescence at 575nm, a fourth fluorescent label which emits its maximum fluorescence at 590nm, a fifth fluorescent label which emits its maximum fluorescence at 650nm, and a sixth fluorescent label which emits its maximum fluorescence at 620nm.

8. The method of item 1, wherein each locus in the set of loci selected is co-amplified using a polymerase chain reaction.

9. The method of item 1, wherein the at least one DNA sample to be analyzed is prepared from human tissue.

10. The method of item 9, wherein the human tissue is selected from one or more of the group consisting of blood, semen, vaginal cells, hair, saliva, urine, bone, buccal sample, amniotic fluid containing placental cells, and amniotic fluid containing fetal cells.

11. A kit comprising oligonucleotide primers for co-amplifying a set of loci of at least one DNA sample to be analyzed; wherein the set of loci can be co-amplified; wherein the primers are in one or more containers; and wherein the set of loci comprises the Amelogenin locus, the 10 STR loci D16S539, D18S51, D19S433, D21S11, D2S1338, D3S1358, D8S1179, FGA, TH01, VWA, and one or more of the group consisting of the STR loci D10S1248, D12S391, D1S1656, D22S1045, and D2S441.

12. The kit of item 11, wherein all of the oligonucleotide primers in the kit are in one container or further comprising reagents for at least one multiplex amplification reaction or further comprising a container having at least one size standard, preferably wherein the size standard is a DNA marker or wherein the size standard is a locus-specific allelic ladder.

13. The kit of item 12, wherein each rung of the locus-specific allelic ladder and at least one oligonucleotide primer for each locus in the set of loci have a fluorescent label covalently attached thereto, and at least two of the oligonucleotide primers have a different fluorescent label covalently attached thereto than other primers in the container.

14. The kit of item 13, wherein at least five of the labeled primers have at least five different fluorescent labels respectively covalently attached thereto, preferably wherein the at least five different fluorescent labels comprise a first fluorescent label which emits its maximum fluorescence at 520nm, a second fluorescent label which emits its maximum fluorescence at 550nm, a third fluorescent label which emits its maximum fluorescence at 575nm, a fourth fluorescent label which emits its maximum fluorescence at 590nm, and a fifth fluorescent label which emits its maximum fluorescence at 650nm.

15. The kit of item 13, wherein at least six of the labeled primers have at least six different fluorescent labels respectively covalently attached thereto, preferably wherein the at least six different fluorescent labels comprise a first fluorescent label which emits its maximum fluorescence at 520nm, a second fluorescent label which emits its maximum fluorescence at 550nm, a third fluorescent label which emits its maximum fluorescence at 575nm, a fourth fluorescent label which emits its maximum fluorescence at 590nm, a fifth fluorescent label which emits its maximum fluorescence at 650nm, and a sixth fluorescent label which emits its maximum fluorescence at 620mn.

**Claims**

1. A method comprising:

   (a) co-amplifying a set of loci from at least one DNA sample to be analyzed, in a multiplex amplification reaction, wherein the product of the reaction is a mixture of amplified alleles from the co-amplified loci in the set, wherein the set of loci includes 15 STR loci D10S1248, D22S1045, D2S441, D12S391, D16S539, D18S51, D19S433, D21S11, D2S1338, D3S1358, D8S1179, FGA, TH01, VWA and D1S1656;
   (b) separating each of the amplified alleles by capillary electrophoresis,

   wherein the co-amplifying step comprises using one pair of oligonucleotide primers for each of the loci in the set of loci, each of the pair of primers flanking a locus of the set of loci in the multiplex reaction, and
   wherein the co-amplifying step comprises using at least four fluorescently labeled oligonucleotide primers, wherein the at least four fluorescently labeled primers have at least four different fluorescent labels respectively covalently attached thereto.

2. The method of claim 1, further comprising co-amplifying the amelogenin locus.

3. The method of claim 2, wherein one primer set each for four of the loci is labeled with a different fluorescent label.

**4.** The method of claim 3, further comprising using five fluorescent labels wherein the fifth fluorescent label is used to label a size standard.

**5.** The method of claim 1 or 2, wherein the co-amplifying step comprises using at least five fluorescently labeled oligonucleotide primers, wherein the at least five labeled primers have at least five different fluorescent labels respectively covalently attached thereto.

**6.** The method of claim 4 or 5, wherein the at least five different fluorescent labels comprise a first fluorescent label which emits its maximum fluorescence at 520nm, a second fluorescent label which emits its maximum fluorescence at 550nm, a third fluorescent label which emits its maximum fluorescence at 575nm, a fourth fluorescent label which emits its maximum fluorescence at 590mn, a fifth fluorescent label which emits its maximum fluorescence at 650nm.

**7.** The method of claim 1 or 2, wherein the co-amplifying step comprises using at least six fluorescently labeled oligonucleotide primers, wherein the at least six labeled primers have at least six different fluorescent labels respectively covalently attached thereto.

**8.** The method of claim 7, wherein the at least six different fluorescent labels comprise a first fluorescent label which emits its maximum fluorescence at 520nm, a second fluorescent label which emits its maximum fluorescence at 550nm, a third fluorescent label which emits its maximum fluorescence at 575nm, a fourth fluorescent label which emits its maximum fluorescence at 590nm, a fifth fluorescent label which emits its maximum fluorescence at 650nm, and a sixth fluorescent label which emits its maximum fluorescence at 620nm.

**9.** The method of claim 3, wherein the amplified alleles for the loci D10S 1248, VWA, D16S539 and D2S1338 are labeled with a different fluorescent label to the amplified alleles to the loci D8S1179, D21S11, D18S51, D22S1045, D19S433, TH01, FGA, D2S441, D3S1358, D1S1656, D12S391 and the amelogenin locus,

wherein the amplified alleles for the loci D8S1179, D21S11, D18S51 and the amelogenin locus are labeled with a different fluorescent label to the amplified alleles of the loci D10S1248, VWA, D16S539, D2S1338, D22S1045, D19S433, TH01, FGA, D2S441, D3S1358, D1S1656 and D12S391,
wherein the amplified alleles for the loci D22S1045, D19S433, TH01 and FGA are labeled with a different fluorescent label to the amplified alleles of the loci D10S1248, VWA, D16S539, D2S1338, D8S1179, D21S11, D18S51, the amelogenin locus, D2S441, D3S1358, D1S1656 and D12S391,
wherein the amplified alleles for the loci D2S441, D3S1358, D1S1656 and D12S391 are labeled with a different fluorescent label to the amplified alleles of the loci D10S1248, VWA, D16S539, D2S1338, the amelogenin locus, D8S1179, D21S11, D18S51, D22S1045, D19S433, TH01 and FGA.

**10.** The method of claim 9, further comprising using a fifth fluorescent label to label a size standard.

**11.** The method of claim 1 or 2, wherein each locus in the set of loci selected is co-amplified using a polymerase chain reaction.

**12.** The method of claim 1, wherein the at least one DNA sample to be analyzed is prepared from human tissue.

**13.** The method of claim 12, wherein the human tissue is selected from one or more of the group consisting of blood, semen, vaginal cells, hair, saliva, urine, bone, buccal sample, amniotic fluid containing placental cells, and amniotic fluid containing fetal cells.

**14.** A kit for use in co-amplifying multiple loci in a single multiplex reaction comprising:

oligonucleotide primers for simultaneously co-amplifying a set of 16 loci of at least one DNA sample to be analyzed, the set of loci comprising the amelogenin locus, and 15 STR loci including D10S1248, D22S1045, D2S441, D12S391, D16S539, D18S51, D19S433, D21S11, D2S1338, D3S1358, D8S1179, FGA, TH01, VWA, and D1S1656, wherein one primer set each for four of the loci is labeled with a different fluorescent label, the primers thereby labeled with four different fluorescent labels;
and a size standard labeled with a fifth fluorescent label.

**15.** The kit of claim 14, further comprising enzyme for amplification, amplification buffer, divalent cations, dNTPs for strand extension during amplification reaction, and instructions for use.

**FIGURE 1**

FIGURE 2

**FIGURE 3**

Europäisches
Patentamt
European
Patent Office
Office européen
des brevets

# EUROPEAN SEARCH REPORT

Application Number

EP 15 20 3179

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GILL P ET AL: "New multiplexes for Europe-Amendments and clarification of strategic development", FORENSIC SCIENCE INTERNATIONAL, ELSEVIER SCIENTIFIC PUBLISHERS IRELAND LTD, IE, vol. 163, no. 1-2, 10 November 2006 (2006-11-10), pages 155-157, XP027940182, ISSN: 0379-0738 [retrieved on 2006-11-10] * abstract; table 1 * * page 156 * | 1-15 | INV. C12Q1/68 |
| Y | ANONYMOUS: "Core STR Loci used in Human Identity Testing", FORENSIC STR INFORMATION , 2006, XP002517547, Retrieved from the Internet: URL:http://http://cstl.nist.gov/div831/strbase/coreSTRs.htm [retrieved on 2009-02-26] * page 1 * | 1-15 | |
| A | BUTLER JOHN M: "Genetics and genomics of core short tandem repeat loci used in human identity testing", JOURNAL OF FORENSIC SCIENCES, CALLAGHAN AND CO., CHICAGO, IL, US, vol. 51, no. 2, 1 March 2006 (2006-03-01), pages 253-265, XP002485019, ISSN: 0022-1198 * page 254; table 1 * | 1-15 | TECHNICAL FIELDS SEARCHED (IPC) C12Q |

-/--

The present search report has been drawn up for all claims

| Place of search | Date of completion of the search | Examiner |
|---|---|---|
| The Hague | 4 July 2016 | Reuter, Uwe |

EPO FORM 1503 03.82 (P04C01)

Europäisches
Patentamt

European
Patent Office

Office européen
des brevets

## EUROPEAN SEARCH REPORT

Application Number

EP 15 20 3179

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | COBLE M D ET AL.: "Characterization of new MiniSTR loci to aid analysis of degraded DNA", JOURNAL OF FORENSIC SCIENCES, vol. 50, no. 1, 2005, pages 43-53, XP002517548, * page 45 * | 1-15 | |
| A | BUTLER J M ET AL: "THE DEVELOPMENT OF REDUCED SIZE STR AMPLICONS AS TOOLS FOR ANALYSIS OF DEGRADED DNA", JOURNAL OF FORENSIC SCIENCES, CALLAGHAN AND CO., CHICAGO, IL, US, vol. 48, no. 5, 1 September 2003 (2003-09-01), pages 1054-1064, XP008065803, ISSN: 0022-1198 * the whole document * | 1-15 | |

TECHNICAL FIELDS
SEARCHED (IPC)

The present search report has been drawn up for all claims

| Place of search | Date of completion of the search | Examiner |
|---|---|---|
| The Hague | 4 July 2016 | Reuter, Uwe |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4683195 A
- US 4683202 A
- US 7008771 B, JW Schumm
- US 5192659 A
- US 6780588 B, SR Coticone
- US 5599666 A
- US 5674686 A
- US 5783406 A

**Non-patent literature cited in the description**

- **M. PONCE ; L. MICOL.** *NAR,* 1992, vol. 20 (3), 623
- **R. DECORTE et al.** *DNA CELL BIOL.,* 1990, vol. 9 (6), 461-469
- **H. WALLACE.** *EMBO REPORTS,* 2006, vol. 7, S26-S30
- **LA DIXON et al.** *FORENSIC SCI. INT,* 2006, vol. 164 (1), 33-44
- **MD COBLE ; JM BUTLER.** *J. FORENSIC SCI.,* 2005, vol. 50 (1), 43-53
- **P. GILL et al.** *FORENSIC SCI. INT.,* 2006, vol. 156 (2-3), 242-244
- **JW SCHUMM et al.** FOURTH INTERNATIONAL SYMPOSIUM ON HUMAN IDENTIFICATION. Promega Corp, 1993, 177-187
- **H. LI et al.** *HUM. MOL. GENET.,* 1993, vol. 2, 1327
- **MV LAREU et al.** *GENE,* 1996, vol. 182, 151-153
- **RE STAUB et al.** *GENOMICS,* 1993, vol. 15, 48-56
- **V. SHARMA ; M. LITT.** *Hum. MOL. GENET.,* 1992, vol. 1, 67
- **KA MILLS et al.** *HUM. MOL. GENET.,* 1992, vol. 1, 779
- **A. EDWARDS.** *AM. J. HUM. GENET.,* 1991, vol. 49, 746-756
- **MH POLYMEROPOULOS et al.** *NUCLEIC ACIDS RES.,* 1991, vol. 19, 3753
- **CP KIMPTON et al.** *HUM. MOL. GENET.,* 1992, vol. 1, 287
- **MD COBLE ; JM BUTLER.** *J. FORENSIC SCI,* 2005, vol. 50 (1), 43-53
- **WATSON et al.** PROGRESS IN FORENSIC GE-NETICS 7: PROCEEDINGS OF THE 17TH INT'L IS-FH CONGRESS. Elsevier, 02 September 1997, 192-194
- **NJ OLDROYD et al.** *ELECTROPHORESIS,* 1995, vol. 16, 334-337
- **MV LAREU et al.** PROGRESS IN FORENSIC GE-NETICS 7: PROCEEDINGS OF THE 17TH INT'L IS-FH CONGRESS. Elsevier, 02 September 1997, 192-200
- **MD COBLE.** *J. FORENSIC SCI,* 2005, vol. 50 (1), 43-53
- **J. SAMBROOK et al.** MOLECULAR CLONING: A LABORATORY MANUAL. Cold Spring Harbor Lab-oratory Press, 1989, 9.14-9.19
- **S. MILLER et al.** *NUCL. ACIDS RES.,* 1988, vol. 16, 1215
- **PS WALSH et al.** *BIOTECHNIQUES,* 1991, vol. 10, 506-513
- **CT COMEY et al.** *J. FORENSIC SCI.,* 1994, vol. 39, 1254
- **J. BURCKHARDT.** *METHODS AND APPLICA-TIONS,* 1994, vol. 3, 239-243
- **RBE MCCABE.** *PCR METHODS AND APPLICA-TIONS,* 1991, vol. 1, 99-106
- **BY NORDVAG.** *BIOTECHNIQUES,* 1992, vol. 12 (4), 490-492
- **CF BRUNK et al.** *ANAL. BIOCHEM.,* 1979, vol. 92, 497-500
- **JS WAYE et al.** *J. FORENSIC SCI,* 1991, vol. 36, 1198-1203
- **RK SAIKI et al.** *SCIENCE,* 1985, vol. 230, 1350-1354
- **DY KWOH ; TJ KWOH.** *AMERICAN BIOTECHNOL-OGY LABORATORY,* October 1990
- **GT WALKER et al.** *PROC. NATL. ACAD. SCI., U.S.A.,* 1992, vol. 89, 392-396
- AMPFLSTR® IDENTIFILER™ PCR AMPLIFICA-TION KIT USER'S MANUAL. Applied Biosystems, 2001, 1-3
- **H. WENZ et al.** *GENOME RES.,* 1998, vol. 8, 69-80
- **E. BUEL et al.** *J. FORENSIC SCI.,* 1998, vol. 43 (I), 164-170
- **M. CHRISTENSEN et al.** *SCAND. J. CLIN. LAB. IN-VEST,* 1999, vol. 59 (3), 167-177

- **J. SAMBROOK et al.** MOLECULAR CLONING: A LABORATORY MANUAL. Cold Spring Harbor Laboratory Press, 1989, 13.45-13.57
- **JW SCHUMM et al.** PROCEEDINGS FROM THE EIGHTH INTERNATIONAL SYMPOSIUM ON HUMAN IDENTIFICATION. Promega Corporation, 1998, 78-84
- **C. PUERS et al.** *AM. J. HUM. GENET,* 1993, vol. 53, 953-958
- **C. PUERS et al.** *GENOMICS,* 1994, vol. 23, 260-264
- **CR PRIMMER et al.** *MOL. ECOL.,* 1995, vol. 4, 493-498
- **DB GOLDSTEIN et al.** *PROC. NATL. ACAD. SCI, U.S.A.,* 1995, vol. 92, 6723-6727
- **MW BRUFORD et al.** *CURIO BIOL,* 1993, vol. 3, 939-943